# EUROPEAN PATENT SPECIFICATION

(11) **EP 3 895 752 B1**
(45) Date of publication and mention of the grant of the patent: **30.08.2023**
(21) Application number: 21382313.1
(22) Date of filing: 14.04.2021
(51) Int. Cl.: A61M 16/20

(54) **RESPIRATOR VALVE**
ATEMGERÄTEVENTIL
VALVE DE RESPIRATEUR

(30) Priority: 15.04.2020 ES 202030640 U
(43) Date of publication of application: 20.10.2021
(73) Proprietor: Hospital Sant Joan de Deu, 08950 Esplugues de Llobregat Barcelona (ES)
(72) Inventor: PALAU FORTE, Enric, 08950 Esplugues de Llobregat (Barcelona) (ES); VALLS ESTEVE, Arnau, 08950 Esplugues de Llobregat (Barcelona) (ES); PONS ODENA, Martí, 08950 Esplugues de Llobregat (Barcelona) (ES); ALAEZ VASCONCELLOS, Carlos, 08950 Esplugues de Llobregat (Barcelona) (ES); FONT VIZCARRA, Lluís, 08950 Esplugues de Llobregat (Barcelona) (ES); BERMÚDEZ CASTEL, Adrian, 08950 Esplugues de Llobregat (Barcelona) (ES); PENA PÉREZ, Xoel, 08950 Esplugues de Llobregat (Barcelona) (ES); MURCIA LAGUNA, Marc, 08950 Esplugues de Llobregat (Barcelona) (ES)
(74) Representative: Herrero & Asociados, S.L.

(56) References cited:
- CN-A- 107 237 906
- US-A1- 2010 282 253
- US-A1- 2012 012 107
- US-A1- 2012 012 111

## Description

The present invention relates to a valve for respirators, ventilators, resuscitators and CPAP (Continuous Positive Airway Pressure) systems in particular, to a PEEP (Positive end-expiratory pressure) valve.

### Background of the invention

PEEP (Positive end-expiratory pressure) valves are valves used in respirators and have a large diameter channel that is closed by means of a disc. A cap forms the top of the valve and the tension from one or two springs is transmitted to a plunger.

When the cap is screwed inward, the spring tension increases. This increase in spring tension in turn results in an increase in the force with which the plunger has to be displaced.

Numerous devices or means are also known for performing PEEP ventilation with the aim of improving the oxygen saturation of arterial blood by increasing oxygen diffusion and preventing the collapse of the smaller airways. This pressure increase in the final phase of expiration can improve arterial oxygenation during ventilation of patients.

In this sense, devices being disposable or with the capacity to be sterilized and reused, with a membrane or without a membrane, having different pressure ranges, with fixed or adjustable pressure, being portable or fixed, can be mentioned.

Currently, diaphragm valves are the only reusable ones. Non-diaphragm valves that are on the market are disposable.

Disposable valves have the disadvantage that they are manufactured with injection plastic in a mold, losing precision in the threaded parts.

Valves that may allow a pressure range high enough to perform the so-called alveolar recruitment maneuver are not known. This maneuver is performed in patients with early acute respiratory distress syndrome.

Normally this maneuver is performed with very sophisticated mechanical ventilation machinery, but not in field respirators. Due to the COVID situation, it is anticipated that this maneuver may be required with hood ventilators.

US2012012111A1 discloses a PEEP valve including a filter media in the air flow between a patient interface and exit vent(s). The patient interface is connected to a patient airway system and the exit vents exhaust exhalation gasses into the atmosphere. The filter prevents or reduces the passage of microbes from the patient's exhalation gasses into the atmosphere. The PEEP valve provides positive gas pressure to a patient's lungs, requiring a predetermined exhalation gas pressure to be exceeded before releasing exhalation gasses into the atmosphere.

### Description of the invention

Therefore, an objective of the present invention is to provide a respirator valve, in particular a portable PEEP valve compatible with hood ventilators, respirators, resuscitators and CPAP systems that may allow increasing values of PEEP and inspiratory pressure, until the pressure mean airway reaches 40 mbar gradually and for a very short time.

With the respirator valve of the invention, the aforementioned drawbacks are solved, presenting other advantages that will be described below.

The respirator valve according to the present invention is defined in claim 1, and it comprises:
- a body provided with a disc;
- an adjustment knob attached to said body;
- a cylindrical spring placed between said adjustment knob and the body disc; and
- a conical spring also placed between said adjustment knob and the body disc, wherein the body and adjustment knob are made of a first sterilizable material and the cylindrical and conical springs are made of a second sterilizable material.

According to a preferred embodiment, said first sterilizable material is polyoxymethylene (POM) and said second sterilizable material is shape memory stainless steel.

Preferably, said conical spring is positioned with its smaller diameter end in contact with the disc, and furthermore the disc comprises a stem positioned centrally on the disc, the conical spring being placed around said stem.

The adjustment knob preferably further comprises an inner shaft provided with a housing to house one end of said stem.

Advantageously, one end of the cylindrical spring is in contact with the upper inner part of the adjustment knob and the other end of the cylindrical spring is in contact with the disc.

Furthermore, the larger diameter end of the conical spring is advantageously in contact with the end of the inner shaft of the adjustment knob closest to the disc.

According to a preferred embodiment, the adjustment knob and the body are attached to each other by respective threads, and the respirator valve according to the present invention also comprises a safety ring placed around the coupling area between the adjustment knob and the body, which allows the valve not to be disassembled during operation.

The respirator valve according to the present invention has the following advantages, among others:
- Optimized design so that it can be manufactured in record time without the need for molds or injection plastic.
- Scalable production capacity, which allows the use of other subcontractors with CNC machinery.
- Easy assembly.
- Functionality and high precision
- Reusable after sterilization.
- Design without membrane, but reusable up to 5 times in autoclave sterilization and up to 50 times in low temperature sterilization with ethylene oxide (EtO) or formaldehyde
- Ergonomic improvement of the "adjustment knobs" so that they do not slip with the gloves of the health personnel that are moistened with disinfectant gel.
- It has an effective cost, since it does not have a membrane.
- Adjustable trigger pressure range from 0 to 20 cm H₂O and alveolar recruitment mode up to 40 cm H₂O.
- Greater hardness when operating the adjustment knob to avoid manipulation errors or false turns compared to current designs.
- V-shaped conical spring arrangement for better precision.
- Springs with heat treatment for shape memory, more resistance to duty cycles.
- Versions with connectors according to ISO with diameters 18 mm, 22 mm and 30 mm.

### Brief description of the drawings

For a better understanding of what has been stated, some drawings are attached in which, schematically and only as a non-limiting example, a practical case of embodiment is represented.
Figure 1 is an exploded perspective view of the components that make up the respirator valve according to the present invention;
Figure 2 is a perspective view of the respirator valve in accordance with the present invention; and
Figure 3 is a sectional elevation view of the respirator valve in accordance with the present invention

### Description of a preferred embodiment

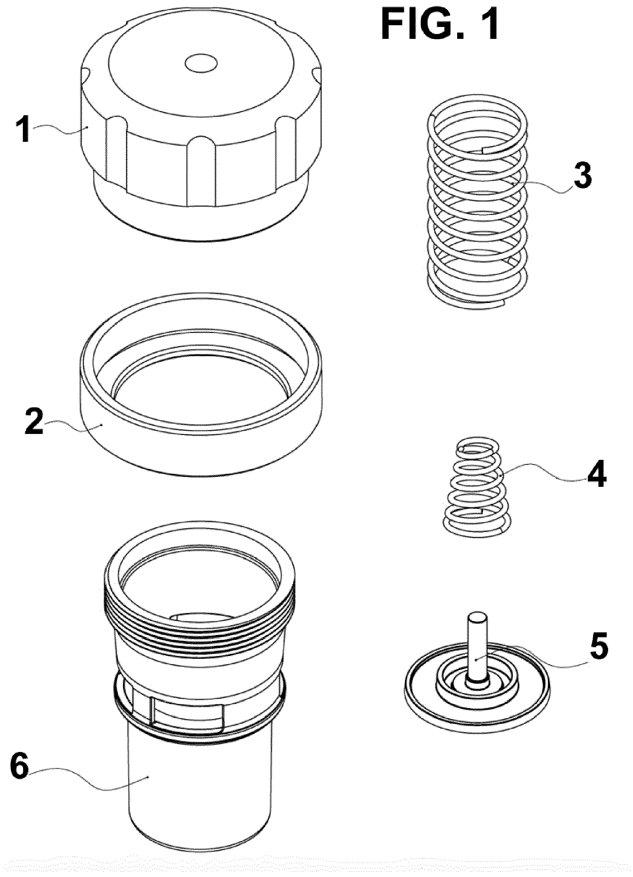

As shown in figure 1, the respirator valve according to the present invention comprises the following elements:
- a body 6 made of a material that allows its sterilization, such as, for example, polyoxymethylene (POM), and that is open at its ends, one of whose ends serves to be attached to a respirator;
- an adjustment knob 1 attached to said body 6 by means of a thread, also made of a material that allows its sterilization, such as, for example, polyoxymethylene (POM);
- a safety ring 2 placed in the coupling zone between the body 6 and the adjustment knob 1, also made of a material that allows its sterilization, such as, for example, polyoxymethylene (POM);
- a cylindrical spring 3 placed between the body 6 and the adjustment knob 1, as will be explained below, and made of a material that allows its sterilization, such as, for example, heat-treated shape memory stainless steel;
- a conical spring 4 placed between the body 6 and the adjustment knob 1, as will be explained below, and made of a material that allows its sterilization, such as, for example, heat-treated shape memory stainless steel; and
- a disc 5 mounted inside the body 6, for example, fixed on an inside step of the body 6, and made of a material that allows its sterilization, such as, for example, polyoxymethylene (POM).

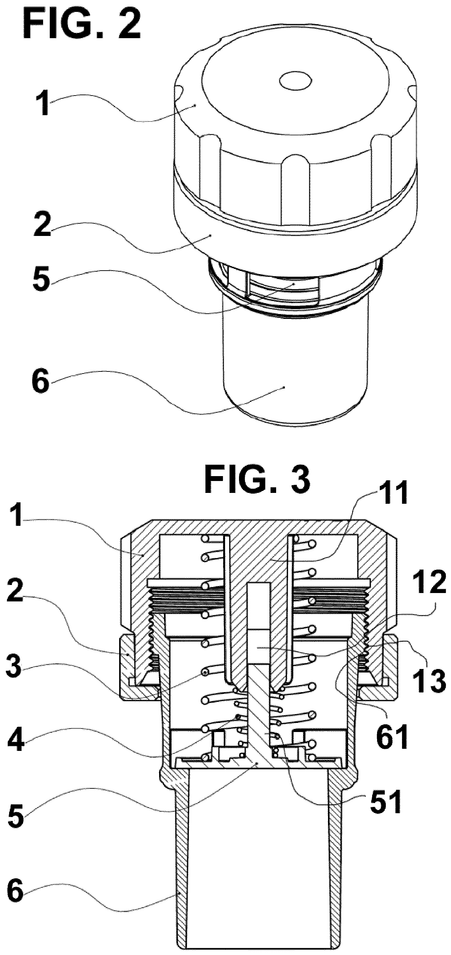

As can be seen in Figures 1 and 3, the disc 5 comprises a stem 51 that protrudes from the disc in a centered position, around which the conical spring 4 is placed in a position in which the part with a smaller diameter is in contact with the disc 5.

For its part, the adjustment knob 1 comprises an inner shaft 11 provided with a housing 12 to house one end of said stem 51 of the disc 5, as shown in figure 3. In this way, the adjustment knob 1 remains perfectly centered with respect to the body 6.

The cylindrical spring 3 is positioned around said inner shaft 11 and said stem 51, between the upper inner part of the adjustment knob 1 and the disc 5, while the conical spring 4 is placed around the stem 5 of the disc, with one of its ends, the end with the largest diameter, in contact with the end of the inner shaft 11 closest to the disc 5.

To adjust the relative position of the adjustment knob 1 with respect to the body 6, and also the compression of the cylindrical 3 and conical springs 4, the adjustment knob 1 comprises an internal thread 13 complementary to an external thread 61 of the body 6, so that the user of the valve can adjust its features by means of said adjustment knob 1.

The operation of the respirator valve according to the present invention is the same as the PEEP valves without membrane, but unlike these it allows its reuse as it is made of materials that can be sterilized and manufactured by computerized numerical control (CNC).

Although reference has been made to a specific embodiment of the invention, it is clear to a person skilled in the art that the respirator valve described is susceptible to numerous variations and modifications, and that all the mentioned details can be technically substituted by others being technically equivalent, without departing from the scope of protection defined by the appended claims.

## Claims

1. Respirator valve, comprising:
- a body (6) provided with a disc (5);
- an adjustment knob (1) attached to said body (6);
- a cylindrical spring (3) positioned between said adjustment knob (1) and the disc (5) of the body (6); and
- a conical spring (4) also placed between said adjustment knob (1) and the disc (5) of the body (6),
**characterized in that** the body (6) and the adjustment knob (1) are made of a first sterilizable material and the cylindrical and conical springs (3, 4) are made of a second sterilizable material,
and **in that** the respirator valve also comprises a safety ring (2) positioned around the coupling zone between the adjustment knob (1) and the body (6).

2. Respirator valve according to claim 1, wherein said first sterilizable material is polyoxymethylene (POM).

3. Respirator valve according to claim 1, wherein said second sterilizable material is shape memory stainless steel.

4. Respirator valve according to claim 1, wherein said conical spring (4) is positioned with its end of smaller diameter in contact with the disc (5).

5. Respirator valve according to claim 1 or 4, wherein the disc (5) comprises a stem (51) positioned centrally on the disc (5), the conical spring (4) being positioned around said stem (51).

6. Respirator valve according to claim 5, wherein the adjustment knob (1) comprises an inner shaft (11) provided with a housing (12) to house one end of said stem (51).

7. Respirator valve according to claim 1, wherein one end of the cylindrical spring (3) is in contact with the upper inner part of the adjustment knob (1) and the other end of the cylindrical spring (3) is in contact with the disc (5).

8. Respirator valve according to claim 6, wherein the end of the larger diameter of the conical spring (4) is in contact with the end of the inner shaft (11) of the adjustment knob (1) closest to the disc (5).

9. Respirator valve according to claim 1, wherein the adjusting knob (1) and the body (6) are attached to each other by means of threads (13, 61).

## Patentansprüche

1. Atemgeräteventil, bestehend aus:
- einen Körper (6), der mit einer Scheibe (5) versehen ist;
- einen Einstellknopf (1), der an dem Körper (6) befestigt ist;
- einer zylindrischen Feder (3), die zwischen dem Einstellknopf (1) und der Scheibe (5) des Körpers (6) positioniert ist; und
- einer konischen Feder (4), die ebenfalls zwischen dem Einstellknopf (1) und der Scheibe (5) des Körpers (6) angebracht ist,
**dadurch gekennzeichnet, dass** der Körper (6) und der Einstellknopf (1) aus einem ersten sterilisierbaren Material bestehen und die zylindrische und die konische Federn (3, 4) aus einem zweiten sterilisierbaren Material bestehen,
und dass das Atemgeräteventil außerdem einen Sicherheitsring (2) umfasst, der um den Kupplungsbereich zwischen dem Einstellknopf (1) und dem Körper (6) herum positioniert ist.

2. Atemgeräteventil nach Anspruch 1, wobei das erste sterilisierbare Material Polyoxymethylen (POM) ist.

3. Atemgeräteventil nach Anspruch 1, wobei das zweite sterilisierbare Material rostfreier Stahl mit Formgedächtnis ist.

4. Atemgeräteventil nach Anspruch 1, wobei die konische Feder (4) mit ihrem Ende kleineren Durchmessers in Verbindung mit der Scheibe (5) positioniert ist.

5. Atemgeräteventil nach Anspruch 1 oder 4, wobei die Scheibe (5) einen mittig auf der Scheibe (5) positionierten Schaft (51) aufweist und die konische Feder (4) um den genannten Schaft (51) herum angebracht ist.

6. Atemgeräteventil nach Anspruch 5, wobei der Einstellknopf (1) eine Innenwelle (11) umfasst, die mit einem Gehäuse (12) versehen ist, um ein Ende des Schaftes (51) aufzunehmen.

7. Atemgeräteventil nach Anspruch 1, wobei ein Ende der zylindrischen Feder (3) in Verbindung mit dem oberen Innenteil des Einstellknopfes (1) und das andere Ende der zylindrischen Feder (3) in Verbindung mit der Scheibe (5) stehen.

8. Atemgeräteventil nach Anspruch 6, wobei das Ende mit dem größeren Durchmesser der konischen Feder (4) in Verbindung mit dem Ende der Innenwelle (11) des Einstellknopfes (1) steht, das der Scheibe (5) am nächsten liegt.

9. Atemgeräteventil nach Anspruch 1, wobei der Einstellknopf (1) und der Körper (6) durch Gewinde (13, 61) aneinander befestigt sind.

## Revendications

1. Valve de respirateur, comprenant :
- un corps (6) muni d'un disque (5) ;
- un bouton de réglage (1) fixé audit corps (6) ;
- un ressort cylindrique (3) positionné entre ledit bouton de réglage (1) et le disque (5) du corps (6) ; et
- un ressort conique (4) également placé entre ledit bouton de réglage (1) et le disque (5) du corps (6),
**caractérisée en ce que** le corps (6) et le bouton de réglage (1) sont faits en un premier matériau stérilisable et les ressorts cylindriques et coniques (3, 4) sont faits en un deuxième matériau stérilisable,
et **en ce que** la valve de respirateur comprend en outre une bague de sécurité (2) positionnée autour de la zone de couplage entre le bouton de réglage (1) et le corps (6).

2. Valve de respirateur selon la revendication 1, dans laquelle ledit premier matériau stérilisable est du polyoxyméthylène (POM).

3. Valve de respirateur selon la revendication 1, dans laquelle ledit deuxième matériau stérilisable est de l'acier inoxydable à mémoire de forme.

4. Valve de respirateur selon la revendication 1, dans laquelle ledit ressort conique (4) est positionné avec son extrémité de plus petit diamètre en contact avec le disque (5).

5. Valve de respirateur selon la revendication 1 ou 4, dans laquelle le disque (5) comprend une tige (51) positionnée centralement sur le disque (5), le ressort conique (4) étant positionné autour de ladite tige (51).

6. Valve de respirateur selon la revendication 5, dans laquelle le bouton de réglage (1) comprend un arbre interne (11) muni d'un logement (12) pour loger une extrémité de ladite tige (51).

7. Valve de respirateur selon la revendication 1, dans laquelle une extrémité du ressort cylindrique (3) est en contact avec la partie interne supérieure du bouton de réglage (1) et l'autre extrémité du ressort cylindrique (3) est en contact avec le disque (5).

8. Valve de respirateur selon la revendication 6, dans laquelle l'extrémité du plus grand diamètre du ressort conique (4) est en contact avec l'extrémité de l'arbre interne (11) du bouton de réglage (1) la plus proche du disque (5).

9. Valve de respirateur selon la revendication 1, dans laquelle le bouton de réglage (1) et le corps (6) sont fixés l'un à l'autre au moyen de filetages (13, 61).
